(19) Europäisches Patentamt
European Patent Office
Office européen des brevets

(11) Publication number: **0 014 796**
**B2**

(12) **NEW EUROPEAN PATENT SPECIFICATION**

(45) Date of publication of the new patent specification: **09.11.88**

(51) Int. Cl.[4]: **C 07 C 45/50,** C 07 F 15/00, B 01 J 31/18

(21) Application number: **79302093.4**

(22) Date of filing: **03.10.79**

(54) **Process for the production of aldehydes by hydroformylation in presence of rhodium compound.**

(30) Priority: **04.10.78 JP 121456/78**
**04.10.78 JP 121457/78**
**06.04.79 JP 40994/79**

(43) Date of publication of application:
**03.09.80 Bulletin 80/18**

(45) Publication of the grant of the patent:
**15.02.84 Bulletin 84/07**

(45) Mention of the opposition decision:
**09.11.88 Bulletin 88/45**

(84) Designated Contracting States:
**BE DE FR GB IT NL**

(56) References cited:
**DE-A-1 793 069**
**DE-A-2 802 922**
**GB-A-1 357 735**
**US-A-3 527 809**
**US-A-3 821 311**
**US-A-3 857 900**

(73) Proprietor: **TOA NENRYO KOGYO KABUSHIKI KAISHA**
**1-1 Hitotsubashi, 1-Chome Chiyoda-Ku**
**Tokyo 100 (JP)**

(72) Inventor: **Sakakibara, Tadamori**
**1902-5 Ooaza Kamekubo Ooi-machi**
**Iruma-gun Saitama-ken (JP)**
Inventor: **Matsushima, Yoshihisa**
**1902-5 Ooaza Kamekubo Ooi-machi**
**Iruma-gun Saitama-ken (JP)**
Inventor: **Nagashima, Yukio**
**1906-199 Ooaza Kamekubo Ooi-machi**
**Iruma-gun Saitama-ken (JP)**
Inventor: **Okamoto, Nobukazu**
**1906-199 Ooaza Kamebuko Ooi-machi**
**Iruma-gun Saitama-ken (JP)**
Inventor: **Kaneko, Katsumi**
**2174-19 Ooaza Nishitsurugaoka Ooi-machi**
**Iruma-gun Saitama-ken (JP)**
Inventor: **Ishii, Yoshio**
**93 Tokugawayama-cho 3-chome Chiskusa-ku**
**Nagoya-shi Aicha-ken (JP)**
Inventor: **Wada, Shozo**
**7-5 Yamanone 2 -chome Zushi-shi**
**Kanagawa-ken (JP)**

(74) Representative: **Bawden, Peter Charles**
**ESSO CHEMICAL LIMITED Esso Chemical Research Centre PO Box 1**
**Abingdon Oxfordshire OX13 6BB (GB)**

**EP 0 014 796 B2**

Courier Press, Leamington Spa, England.

**Description**

This invention relates to the production of aldehydes from olefin, hydrogen and carbon monoxide using a rhodium catalyst system.

Carbonyl tris (triphenylphosphine) rhodium hydride is known as a catalyst having activity in various chemical reactions for organic syntheses, for example, in hydroformylation. (Japanese Patent Publication No. 10730/1970 equivalent to US Patent 3 527 809). See also US Patent 3 821 311 and Canadian Patent 992 101.

Rhodium-phosphine type or rhodium-phosphite type complexes are known as catalysts for the hydroformylation of olefins. The stability of a rhodium catalyst is increased by modification with phosphine, arsine, or stibine, which permits practising the oxo reaction at a rather low pressure.

According to Japanese Patent No. 903,326, straight chain-rich aldehydes are prepared at a low total pressure with a low partial pressure of carbon monoxide and a high partial pressure of hydrogen in the presence of a rhodium triarylphosphine catalyst and a triarylphosphine ligand in a large excess to the rhodium. However, this method has the disadvantage that the hydroformylation reaction rate of an olefin is markedly decreased because of using a ligand in a large excess to rhodium, and a considerable quantity of a paraffin is formed by the hydrogenation of the olefin ("Hydrocarbon Processing" (4) 112 (1970)) due to the reaction at a low total pressure with a low partial pressure of carbon monoxide and a high partial pressure of hydrogen.

Rhodium catalysts in combination with arsines or stibines instead of phosphines have been proposed, but the studies thereof have not been made so intensively because of their lower activity compared with tertiary phosphine-rhodium catalysts.

It has been found that catalyst systems comprising a rhodium source and certain mixed free ligands in excess are useful in hydroformylation reactions. Accordingly, the present invention provides a process for producing an aldehyde by hydrofomrylating an olefin with hydrogen and carbon monoxide in the presence of a rhodium containing hydroformylation catalyst system to form an aldehyde having one more carbon atom than the olefin characterised in that the catalyst system comprises a rhodium source and free mixed ligands in excess of the quantity required for coordination to the rhodium atom, said ligands comprising a tertiary organo phosphorus and a tertiary organo arsenic of formula $X'R''_3$ wherein X' is phosphorus or arsenic and each R'' independently represents an organic group.

Thus the invention relates to the production of aldehydes by the hydroformylation of olefins and more particularly, it is concerned with an improved process for producing aldehydes by reacting olefins other than octene-1, carbon monoxide and hydrogen in the presence of rhodium-containing complex compound catalysts and free ligands. Surprisingly, it has been found that by using a catalyst system comprising rhodium and mixed ligands of tertiary organo phosphorus ligands and tertiary organo arsenic ligands in combination, with excess mixed ligands, the reaction rate is remarkably improved, the quantity of paraffin formed by the hydrogenation of the olefin is decreased and, in addition, the selectivity to normal chain aldehyde is kept similar or improved in comparison with carrying out the reaction in the presence of a tertiary organo phosphorus rhodium catalyst and excess tertiary organo phosphorus ligand. Also, the catalyst can be re-used with maintenance of its activity after separating the prepared aldehyde by suitable means such as distillation and the like.

In particular the invention provides a commercially valuable process whereby hydroformylation of an olefin is improved using a rhodium complex containing both a tertiary organo phosphorus compound and tertiary organo arsenic compound in a rhodium complex $HRh(CO)(ligand)_3$ which is an active species for the hydroformylation in the presence of an excess quantity of mixed ligands or a tertiary organo phosphorus compound and tertiary organo arsenic compound. Included with the scope of the invention is a process for producing aldehydes by reacting an olefin other than octene-1 with carbon monoxide and hydrogen, the aldehydes having one more carbon atom than the olefin, which comprises carrying out the reaction using a rhodium catalyst containing a tertiary organo phosphorus and tertiary organo arsenic represented by the general formula $X'R''_3$ (wherein X' represents phosphorus or arsenic and R'' represents an organic group, in particular, alkyl, cycloalkyl, aryl or aralkyl group, which may be same or different) and carbon monoxide in the presence of free mixed ligands comprising a tertiary organo phosphorus and tertiary organo arsenic represented by the general formula $X'R''_3$ (wherein R'' has the same meaning as above and may be the same as or different from those contained in the above described rhodium catalyst) in excess of the quantity required for coordination to the rhodium atom.

The rhodium catalyst used in the present invention may be prepared by synthesizing a rhodium complex represented by the general formula set forth below and combining this complex with excess mixed ligands. For example, the rhodium complex represented by the general formula:

$$HRh(CO)(PR^1R^2R^3)n(AsR^4R^5R^6)3\text{-}n$$

in which $R^1$, $R^2$, $R^3$, $R^4$, $R^5$ and $R^6$ represent alkyl, cycloalkyl, aryl and aralkyl groups and may be the same or different, and n is 1 or 2, is made by first contacting $RhZ(CO)(PR^1R^2AR^3)_2$ (wherein Z represents a halide anion) with $AsR^4R^5R^6$ in a solvent, followed by gradually adding a reducing agent of a hydride type of Group IIIA elements of the Periodic Table such as sodium borohydride at a relatively low temperature.

Preferably, $R^1$, $R^2$, $R^3$, $R^4$, $R^5$ and $R^6$ are aryl groups and more preferably the same aryl group. In view of the reactivity and commercial availability, it is most preferable that they are all phenyl groups, thus a combination of triphenylarsine and triphenylphosphine. The mixed ligands used in excess may be the same as or different from those contained in the rhodium complex, but the same combinations are advantageous commercially. The ratio or tertiary organo phosphorus to tertiary organo arsenic used as mixed ligands in excess is 20:1 to 1:20, preferably 10:1 to 1:10 and a range of 2:1 to 1:2 is most preferable.

The sum of the amounts of mixed ligands to be added in excess of those capable of combining with a rhodium atom through coordination in a rhodium complex may be chosen within a wide range such that the mixed ligands-containing complex is substantially present. In general, the sum of the amounts of free mixed ligands is 3 mols or mols or more per rhodium atom in the mixed ligands-containing rhodium complex. Since addition of free mixed ligands in too large an excess is disadvantageous with respect to the reaction and cost, the excess amount is preferably 5 to 1000 mols, preferably 50 to 500 mols per rhodium atom.

As an alternative to using a previously prepared rhodium complex, an oxo catalyst can be formed in situ, i.e. under hydroformylation conditions. That is to say, it is possible to use a method comprising contacting a rhodium metal such as rhodium black or supported rhodium, an inorganic rhodium compound such as rhodium oxide or rhodium nitrate, an organic acid salt such as rhodium acetate or rhodium octoate, or a rhodium complex such as rhodium carbonyl, rhodium dicarbonyl acetylacetone or rhodium carbonyl triphenylphosphine acetylacetone, in the presence of the above described mixed ligands with a mixed gas of carbon monoxide and hydrogen at elevated temperature before or during hydroformylation.

When a hydroformylation reaction of an olefin is carried out using a catalyst according to this invention, the reaction rate is increased to 1.5 to 2 times as much as when a single ligand of tertiary organo phosphorous is used and, accordingly, a reactor can be reduced in size with the same output. Furthermore, it is possible to decrease the quantity of rhodium catalyst to olefin and the concentration of rhodium in a catalyst bed.

The quantity of rhodium catalyst used in a hydroformylation, depending on the procedure and the variety of olefin used as a starting material, may be chosen — considering the conditions — within a wide range. In a catalyst recycling process wherein an olefin, synthesis gas and rhodium catalyst are fed to a reaction tower, the reaction mixture is withdrawn from the head of the tower, cooled and subjected to reduced pressure to separate a gaseous component, and the liquid produced is passed through a distillation column to distill off the product and to give a still residue containing the rhodium catalyst which is then withdrawn from the bottom of the column and recirculated to the reaction tower, for example, the quantity of the rhodium catalyst may be 10 ppm to 5% by weight, preferably 50 ppm to 1% by weight as rhodium metal based on olefin feed. In a liquid fixed bed process wherein an olefin and synthesis gas are fed to a catalyst layer charged previously to a reaction tower and only the reaction product in the form of a mixture of gases and vapors is withdrawn from the top of the tower, the concentration of rhodium in the catalyst layer is generally 10 ppm to 5% by weight, preferably 50 ppm to 1% by weight.

The catalyst system of the present invention may be used batchwise in addition to the continuous procedures described above and is also applicable to liquid, vapor or mixed phase processes. The reaction conditions may be the same as those in the case of using the rhodium-tertiary organo phosphorus type catalysts. That is to say, the reaction temperature is ordinarily room temperature to about 150°C, preferably 50 to 130°C, most preferably 80 to 120°C and the total pressure is ordinarily normal pressure to about 100 atmospheres, preferably normal pressure to 50 atmospheres, most preferably normal pressure to 30 atmospheres. In addition the hydrogen to carbon monoxide molar ratio is 10/1 to 1/10, preferably 10/1 to 1/1. A solvent is not always indispensable, but in order to maintain stable operation, it is desirable to use a solvent.

The solvent may be chosen from a wide range among those having no detrimental influence on hydroformylation, for example, saturated hydrocarbons such as hexane, decane and dodecane; aromatic hydrocarbons such as benzene, toluene, xylene, cumene and diisopropylbenzene; and oxygen-containing compounds such as alcohols, ketones, esters and preferably products and high boiling point by-products of hydroformylation.

The catalyst system of the present invention can be adapted to α-olefins, such as ethylene, propylene, butene-1 and hexene-1, olefins with interal double bonds such as butene-2 and octene-2, and vinly compounds such as styrene, acrylonitrile, acrylic acid esters and allyl alcohols. The present catalyst system is most suitable for obtaining aldehydes rich in normal chain type isomers from α-olefins.

Certain novel rhodium compounds, and a process for their production, are described and claimed in copending European patent application , divided out of the present application. Such compounds have the general formula

$$HRh(CO)(XR_3)_n(YR'_3)_{3-n}$$

in which X is phosphorus, arsenic or antimony; Y is arsenic or antimony when X is phosphorus, Y is phosphorus or antimony when X is arsenic, or Y is phosphorus, arsenic or antimony when X is antimony; R and R′ are aryl groups and n is an integer of 1 or 2. These compounds, without the use of excess mixed

ligands according to the present invention, have proved to be useful as hydroformylation catalysts; data on these novel compounds are included herein for comparison to illustrate the present invention by Example.

Comparative Example 1

20 ml of n-dodecane and 0.109 mmol of a novel compound as mentioned above, prepared as described in Example 1 of copending divisional European Patent Application were charged to a 300 ml stainless steel autoclave, which was purged with nitrogen. 5.0 g (119 mmol) of propylene was introduced under pressure into the autoclave which was then heated to 110°C, and $H_2/CO$ gas with a molar ratio of 1/1 being introduced, and the reaction was carried out with the reaction pressure maintained at 20 $Kg/cm^2$. The time when the $H_2/CO$ gas was no longer absorbed was regarded as the final point of the reaction. After the reaction, the reaction product was subjected to analysis by gas chromatagraphy, giving the results shown in Table 1.

It is apparent from the results of Table 1 that the rhodium compounds used, even without an excess of mixed ligands in the catalyst system are very effective as a catalyst for the oxo reaction.

TABLE 1

| Rhodium compound | Reaction rate $-d(H_2+CO)/dt$ (ml/sec) | Reaction time (min) | Conversion of propylene (mol %) | Yield of butylaldehyde (mol %) | n/i Ratio of butylaldehyde | Yield of propane (mol %) |
|---|---|---|---|---|---|---|
| $HRh(CO)(AsPH_3)(PPh_3)_2$ | 13.5 | 13 | 90.8 | 89.8 | 2.0 | 0.7 |
| $HRh(CO)(SbPh_3)(PPh_3)_2$ | 1.5 | 100 | 43.0 | 42.1 | 1.9 | 0.4 |
| $HRh(CO)(PPh_3)(AsPh_3)_2$ | 10.2 | 30 | 89.5 | 88.6 | 2.1 | 0.6 |
| $HRh(CO)(SbPh_3)(AsPh_3)_2$ | 0.5 | 300 | 19.7 | 18.3 | 2.1 | 0.2 |
| $HRh(CO)(PPh_3)(SbPh_3)_2$ | 1.2 | 275 | 50.4 | 49.8 | 2.0 | 0.4 |
| $HRh(CO)(AsPh_3)(SbPh_3)_2$ | 0.4 | 300 | 17.7 | 16.8 | 2.2 | 0.3 |
| $HRh(CO)(SbPh_3)_3$ | 0.2 | 300 | 7.5 | 7.0 | 2.3 | 0.3 |

Example 1

Comparative Example 1 was essentially followed with the modification that in addition to 0.109 mmol of the complex $HRh(CO)(PPh_3)_2(AsPh_3)$, triphenylphosphine and triphenylarsine were charged to the autoclave respectively in a proportion of 50 mols to 1 mol of the rhodium complex. They synthesis gas was continuously supplied from a gas holder to keep the reaction pressure constant. After the reaction had been continued for 22 minutes, from the start until the conversion of propylene reached approximately 90%, the autoclave was cooled rapidly and the product was withdrawn and subjected to analysis by gas chromatography. The reaction rate measured by the pressure decrease of the synthesis gas in the gas holder was 8.8 ml/sec. The conversion of propylene was 87.2% and the ratio of normal isomer to branched isomer was 3.7. The quantity of propane formed by the hydrogenation of the propylene was 0.4 mol%.

Examples 2 to 7

Hydroformylations using as catalyst the complexes

$$Hrh(CO)(PPh_3)_2(AsPh_3) \text{ and } HRh(CO)(PPh_3)(AsPh_3)_2$$

as in Comparative Example 1 were carried out in a manner analogous to Example 1 but with variation of the quantity of excess ligands and reaction conditions, thus obtaining the results shown in Table 2.

Comparative Examples 2 to 6

Hydroformylations using as catalyst the complex $HRh(CO)(PPh_3)_3$ synthesized in conventional manner were carried out in a manner analogous to Example 1 with change of conditions as shown in Table 2, thus obtaining the results shown therein.

As can be seen from the Examples and the Comparative Examples, when an oxo resaction is carried out using as catalyst the complex containing mixed ligands and the excess ligands, the reaction rate is increased to about 1.5 to 2 times as much as that obtained when using the complex containing phosphine only and the excess phosphine only, the normal chain to branched chain ratio of butyaldehyde is somewhat increased and the quantity of propane formed is somewhat decreased.

Example 8

The hydroformylation reaction described in Example 1 was essentially repeated except that there was used as catalyst a still residue containing the rhodium catalyst and excess mixed ligands obtained by distilling the product of the hydroformylation reaction of Example 2 and separating the aldehyde. The results obtained are shown in Table 2, from which it is apparent that the activity and selectivity are not changed and thus the catalyst can be reused even after a heat treatment such as distillation.

Example 9

The hydroformylation of Example 2 was repeated except that there was used a catalyst consisting of rhodium supported on activated carbon (Rh content: 1.0% by weight) and excess mixed ligands, thus obtaining the results shown in Table 2.

TABLE 2

| Ex. No. | Rhodium complex (0.109 mmol) | $PPh_3$/Rh complex (mol ratio) | $AsPh_3$/Rh complex (mol ratio) | Reaction tempera-ture (°C) | $H_2$/Co (mol ratio) | Reaction rate $-d(H_2/CO)/dt$ (ml/sec) | Propylene conversion (mol %) | Butylaldehyde normal/ branched ratio | Propane yield (mol %) |
|---|---|---|---|---|---|---|---|---|---|
| 1 | $HRh(CO)(PPh_3)_2(AsPh_3)$ | 50 | 50 | 110 | 1.0 | 8.8 | 87.2 | 3.7 | 0.4 |
| 2 | ″ | 67 | 33 | 110 | 1.0 | 7.0 | 89.3 | 3.9 | 0.2 |
| 3 | ″ | 100 | 50 | 110 | 1.0 | 6.0 | 89.1 | 4.5 | 0.3 |
| 4 | ″ | 67 | 33 | 90 | 1.0 | 1.8 | 87.5 | 3.9 | 0.2 |
| 5 | ″ | 100 | 50 | 110 | 5.0 | 7.6 | 88.1 | 8.2 | 1.8 |
| 6 | ″ | 67 | 33 | 110 | 5.0 | 9.1 | 92.9 | 6.4 | 1.6 |
| 7 | $HRh(CO)(PPh_3)(AsPh_3)_2$ | 33 | 67 | 110 | 1.0 | 12.6 | 88.7 | 3.5 | 0.4 |
| 2* | $HRh(CO)(PPh_3)_3$ | 67 | 0 | 110 | 1.0 | 4.4 | 86.4 | 3.4 | 0.3 |
| 3* | ″ | 100 | 0 | 110 | 1.0 | 4.0 | 90.2 | 4.0 | 0.7 |
| 4* | ″ | 100 | 0 | 110 | 5.0 | 5.5 | 90.5 | 6.6 | 2.8 |
| 5* | ″ | 33 | 0 | 110 | 1.0 | 5.8 | 89.5 | 2.7 | 0.6 |
| 6* | ″ | 100 | 0 | 90 | 1.0 | 1.0 | 87.2 | 4.0 | 0.4 |
| 8 | Catalyst of Ex. 2 (reused) | — | — | 110 | 1.0 | 6.8 | 90.2 | 3.9 | 0.2 |
| 9 | Rh/C (Rh 1.0 wt.%) | 67 | 33 | 110 | 1.0 | 6.0 | 88.2 | 3.8 | 0.2 |

Note: Total pressure=20 $Kg/cm^2$
*Comparative Example

In addition to using the novel compounds exemplified hereinbefore as components of the catalyst system which characterises the present invention, it is possible to use other rhodium sources in conjunction with the excess mixed ligands. Thus in another of its aspects, the present invention has advantages with regard to the ease of preparing or providing a rhodium-mixed ligands catalyst.

The inventors have made studies of a catalyst system consisting of an easily synthesizable or available rhodium source, for example, an inorganic rhodium compound such as rhodium metal or rhodium oxide, a rhodium organic salt such as rhodium acetate, or a rhodium complex such as rhodium carbonyl, $Rh(CO)(PPh_3)_2Cl$ or $HRh(CO)(PPh_3)_3$ and excess mixed ligands of a tertiary organo phosphorus and tertiary organo arsenic and have found that when hydroformylation is carried out with this catalyst system, the effect of hydroformylation is equal to that of the catalyst described in the foregoing.

The easily obtainable or easily prepared rhodium source which may be employed in the process of the invention includes various rhodium compounds.

Examples are a rhodium metal such as rhodium black or supported rhodium, an inorganic rhodium compound such as rhodium oxide, rhodium nitrate, rhodium sulfate, rhodium chloride, rhodium bromide or rhodium iodide, a rhodium organic acid salt such as rhodium acetate or rhodium octoate, or a rhodium complex such as rhodium carbonyl,

$Rh(CO)_2(acac)$,
$Rh(CO)(acac)(PPh_3)$,
$Rh(CO)(PPh_3)_2Cl$,
$[Rh(CO)_2Cl]_2$,
$HRh(CO)(PPh_3)_3$,
$HRh(CO)(AsPh_3)_3$,
$HRh(CO)[P(OPh)_3]_3$,
$Rh(acac)_3$, $RhCl(PPh_3)_3$,
$[RhCl(C_2H_4)_2]_2$,
$[RhCl(1,5\text{-}COD)]_2$,
$Rh(CO)(AsPh_3)_2Cl$,
$Rh(NO)(PPh_3)_3$,
$[(1,5\text{-}COD)Rh(PPh_3)_2]ClO_4$ or
$[(1,5\text{-}COD)Rh(AsPh_3)_2]ClO_4$.

(wherein acac=acetylacetonate, Ph=phenyl and COD=cyclooctadienyl).

Preferred examples from the viewpoint of commercial availability and ease of preparation are complex compounds such as

$HRh(CO)(PPh_3)_3$,
$HRh(CO)(AsPh_3)_3$,
$Rh(CO)_2(acac)$,
$[Rh(PPh_3)_3]_2$,
$HRh(Ph_2PCH_2CH_2PPh_2)_2$ and
$HRh(CO)[P(OPh)_3]_3$.

The ligands $(X'R''_3)$ can have the general formula $PR^7R^8R^9$ and $AsR^{10}R^{11}R^{12}$ in which $R^7$ to $R^{12}$ represent alkyl, cycloalkyl, aryl, aralkyl, alkoxy, cycloalkoxy, aryloxy, and aralkyloxy groups and may be the same or different. Preferably, $R^7$, $R^8$, $R^9$, $R^{10}$, $R^{11}$ and $R^{12}$ are aryl groups and aryloxy groups and more preferably the same aryl group. In view of the reactivity and commercial availability, it is most preferable that they are all phenyl groups, that is, triphenyl phosphine and triphenyl arsine. The mixed ligands used in excess may be the same as or different from those contained in the rhodium complex, but the same ligands are advantageous commercially.

As with the previously exemplified rhodium sources the sum of the amounts of mixed ligands may be chosen within a wide range. In general, the sum of the amounts of free mixed ligands is 3 mols or more per rhodium atom in the rhodium source. Since addition of free mixed ligands in too large an excess is disadvantageous with respect to the reaction and cost, the excess amount is preferably 5 to 1000 mols, most preferably 50 to 500 mols per rhodium atom.

The proportion of tertiary organo phosphorus to tertiary organo arsenic used as mixed ligands in excess may be chosen within a wide range. This proportion is generally 5:1 to 1:5, preferably 2:1 to 1:3, in order to maintain the stability of the catalyst and show the effect of a mixed ligands system sufficiently.

The above mentioned rhodium souce and mixed ligands are provided in a hydroformylation reactor in order to carry out the hydroformylation but the rhodium source may be contacted with a mixed gas of carbon monoxide and hydrogen in the presence of the mixed ligands in excess at an elevated pressure before the hydroformylation. In particular, a rhodium source containing halogen or rhodium nitrate is preferably treated with an alkaline reactant, e.g., aqueous sodium hydroxide, outside the reaction system under the above mentioned conditions.

It is known that the hydroformylation rate decreases with increasing mol ratio of a tertiary organo phosphorus to rhodium when carrying out the hydroformylation in the presence of a tertiary organo phosphorus-rhodium complex and a tertiary organo phosphorus in excess, see Journal of the Chemical Society (A) 1970, pp. 2753—2764.

On the other hand, in a hydroformylation in the presence of a tertiary organo phosphorus-rhodium complex and a tertiary organo arsenic in excess, decrease of the reaction rate is observed too, with increasing mol ratio of a tertiary organo arsenic to rhodium, and moreover, the rate is extremely slow and the selectivity to normal aldehyde is low compared with that in the tertiary organo phosphorus in excess.

The present invention shows the unique and commercially valuable result, which comprises not slowing down the rate of hydroformylation and not lowering the selectivity to n-butylaldehyde caused by the presence of a tertiary organo arsenic in excess, but maintaining the selectivity to normal-butylaldehyde and improving the reaction rate to 1.5 to 2 times that of the known process using tertiary phosphorus ligands.

The present invention provides a commercially valuable process whereby in the hydroformylation of olefins, the reaction rate is increased, the quantity of paraffin by-product is decreased and, in addition, the selectivity to normal chain aldehyde is kept similar or improved in comparison with carrying out the reaction in the presence of a rhodium-tertiary organo phosphorus complex and excess tertiary organo phosphorus ligand in combination; and the greater ease of obtaining the catalyst and the similar effect on reaction rate, by-product and selectivity to normal aldehyde are shown in comparison with carrying out the reaction in the presence of a rhodium-tertiary organo phosphorus + tertiary organo arsenic complex and excess mixed ligands of tertiary organo phosphorus + tertiary organo arsenic ligand.

The following examples are given in order to illustrate the present invention in detail without limiting the same, using rhodium souces other than those novel compounds exemplified hereinbefore.

Example 10

0.109 mmol of tris(triphenyl phosphine) rhodium carbonyl hydride [$HRh(CO)(PPh_3)_3$], 3.60 mmol of triphenyl phosphine, 3.60 mmol of triphenylarsine and 20 ml of n-dodecane were charged to a 300 ml stainless steel autoclave equipped with a magnetic stirrer which was purged with nitrogen. 5.0 g of propylene was introduced under pressure into the autoclave which was then heated to 110°C, an $H_2/CO$ gas with a molar ratio of 1:1 was introduced and the reaction pressure was adjusted to 20 Kg/cm$^2$, followed by stirring. The synthesis gas was continuously supplied from a gas holder to keep the reaction pressure constant. After the reaction had been continued for 18 minutes, from the start until the conversion of propylene reached about 90%, the autoclave was cooled and the product was withdrawn and subjected to analysis by gas chromatography. The reaction rate measured by the pressure decrease of the synthesis gas in the gas holder was 11.1 ml/sec. The conversion of propylene was 89.3% and the ratio of normal isomer to branched isomer of butylaldehyde was 3.1. The yield of propane was 0.4%.

Examples 11 to 18 and Comparative Examples 7 to 12

Hydroformylations were carried out in a manner analogous to Example 10 except that the ratio of $PPh_3$/rhodium and $AsPH_3$/rhodium, reaction temperature and the mol ratio of $H_2/CO$ were varied, thus obtaining the results shown in the following Table 3.

As can be seen from these results, in the catalyst system containing mixed excess $PPh_3$ and $AsPH_3$, the reaction rate is increased to about 1.5 to 2 times that in the catalyst system containing excess $PPh_3$ only and the quantity of propane formed is somewhat decreased.

Example 19 and Comparative Example 13

$RhCl(CO)(AsPh_3)_2$ which was synthesized from $RhCl_3 \cdot 3H_2O$ by the known method [L. Vallarince, J. Chem. Soc., (A)2287 (1966)] was reacted in the presence of $AsPh_3$ in excess, in ethanol solvent and under a nitrogen atmosphere at 65°C. After the reaction, the mixture was cooled to about 0°C, and then an ethanol solution of $NaBH_4$ was added dropwise, thereby forming $HRh(CO)(AsPh_3)_3$.

Hydroformylations were carried out in a manner analogous to Example 10 except that the abovementioned complex as rhodium source and mixed excess ligands of 5.45 mmol $PPh_3$ and 5.45 mmol $AsPh_3$ were used. The results are shown in Table 3 with the results of Comparative Example 13 carried out in the presence of $AsPh_3$ as the only excess ligand.

It is apparent from these results that in a $HRh(CO)(AsPh_3)_3/AsPh_3$ catalyst system, the activity and the selectivity to normal chain aldehyde are lower, but in a mixed ligands catalyst system, higher activity and higher selectivity are achieved.

Example 20

$Rh(CO)_2(CH_3COCH_2COCH_3)$ was synthesized from $[Rh(CO)_2Cl]_2$ by the known method [B. E. North et al. J. Organometal Chem., *21* 445 (1970)]. Hydroformylations were carried out in a manner analogous to Example 10 except that this complex as rhodium source and mixed ligands of 5.45 mmol $PPh_3$ and 5.45 mmol $AsPh_3$ were used. The results are shown in Table 3.

Example 21

The hydroformylation of Example 10 was repeated except that a catalyst of rhodium-mixed ligands and excess ligands was used. It is apparent from Table 3 that the other Examples and Example 21 show a similar effect on activity and selectivity to normal chain aldehyde.

TABLE 3

| Example No. | Rhodium source (0.109 mmol) | $PPh_3$/Rh source (mol ratio) | $AsPh_3$/Rh source (mol ratio) | $AsPh_3/PPh_3$ (mol ratio) | Reaction temperature (°C) | $H_2$/CO (mol ratio) | Reaction rate $-d(H_2/CO)/dt$ (ml/sec) |
|---|---|---|---|---|---|---|---|
| 10 | $HRh(CO)(PPh_3)_3$ | 33 | 33 | 1.0 | 110 | 1/1 | 11.1 |
| 11 | ,, | 33 | 67 | 2.0 | 110 | 1/1 | 12.8 |
| 12 | ,, | 50 | 50 | 1.0 | 110 | 1/1 | 8.4 |
| 13 | ,, | 50 | 75 | 1.5 | 110 | 1/1 | 8.6 |
| 14 | ,, | 50 | 150 | 3.0 | 110 | 1/1 | 6.9 |
| 15 | ,, | 100 | 50 | 0.5 | 110 | 1/1 | 5.8 |
| 16 | ,, | 100 | 50 | 0.5 | 100 | 1/1 | 3.2 |
| 17 | ,, | 100 | 50 | 0.5 | 110 | 5/1 | 7.5 |
| 18 | ,, | 67 | 33 | 0.5 | 110 | 1.0 | 4.4 |
| Comparative Example | | | | | | | |
| 7 | $HRh(CO)(PPh_3)_3$ | 33 | — | — | 110 | 1/1 | 5.8 |
| 8 | ,, | 50 | — | — | 110 | 1/1 | 5.6 |
| 9 | ,, | 100 | — | — | 110 | 1/1 | 4.0 |
| 10 | ,, | 200 | — | — | 110 | 1/1 | 2.5 |
| 11 | ,, | 100 | — | — | 100 | 1/1 | 2.3 |
| 12 | ,, | 100 | — | — | 110 | 5/1 | 5.5 |
| Example | | | | | | | |
| 19 | $HRh(CO)(AsPh_3)_3$ | 50 | 50 | 1.0 | 110 | 1/1 | 7.9 |
| Comparative Example | | | | | | | |
| 13 | ,, | — | 50 | — | 110 | 1/1 | 1.0 |
| Example | | | | | | | |
| 20 | $Rh(CO)_2(acac)$ | 50 | 50 | 1.0 | 110 | 1/1 | 7.6 |
| 21 | $HRh(CO)(PPh_3)_2(AsPh_3)$ | 50 | 50 | 1.0 | 110 | 1/1 | 8.8 |

TABLE 3 (Cont.)

| Example No. | Rhodium source (0.109 mmol) | Propylene conv. (mol %) | n/i of butylaldehyde (mol ratio) | Propane yield (mol %) |
|---|---|---|---|---|
| 10 | $HRh(CO)(PPh_3)_3$ | 89.3 | 3.1 | 0.4 |
| 11 | " | 90.2 | 3.3 | 0.4 |
| 12 | " | 87.2 | 3.5 | 0.3 |
| 13 | " | 89.8 | 3.6 | 0.4 |
| 14 | " | 87.2 | 3.8 | 0.4 |
| 15 | " | 87.3 | 4.3 | 0.3 |
| 16 | " | 93.9 | 4.3 | 0.2 |
| 17 | " | 90.4 | 7.9 | 1.9 |
| 18 | " | 82.3 | 3.6 | 0.2 |
| Comparative Example | | | | |
| 7 | $HRh(CO)(PPh_3)_3$ | 89.6 | 2.7 | 0.7 |
| 8 | " | 90.9 | 2.9 | 0.7 |
| 9 | " | 90.2 | 4.0 | 0.5 |
| 10 | " | 88.7 | 5.2 | 0.4 |
| 11 | " | 87.1 | 4.0 | 0.5 |
| 12 | " | 90.4 | 6.6 | 2.3 |
| Example | | | | |
| 19 | $HRh(CO)(AsPh_3)_3$ | 90.7 | 3.3 | 0.3 |
| Comparative Example | | | | |
| 13 | " | 49.8 | 1.7 | 0.3 |
| Example | | | | |
| 20 | $Rh(CO)_2(acac)$ | 86.5 | 3.4 | 0.4 |
| 21 | $HRh(CO)(PPh_3)_2(AsPh_3)$ | 87.2 | 3.7 | 0.4 |

**Claims**

1. A process for producing an aldehyde by hydroformylating an olefin other than octene-1 with hydrogen and carbon monoxide in the presence of a rhodium containing hydroformylation catalyst system to form an aldehyde having one more carbon atom than the olefin characterised in that the catalyst system comprises a rhodium souce and free mixed ligands in excess of the quantity required for coordination to the rhodium atom, said ligands comprising a tertiary organo phosphorus and a tertiary organo arsenic of formula $X'R''_3$ wherein X' is phosphorus or arsenic and each R'' independently represents an organic group, the mixed tertiary organo phosphorus and tertiary organo arsenic ligands being in a mol ratio of from 20:1 to 1:20.

2. A process according to claim 1 in which the rhodium source is selected from the group consisting of rhodium metal, an inorganic rhodium compound, a rhodium salt of an organic acid and a rhodium complex containing any of the following ligands:

(a) CO
(b) acetylacetonate
(c) cyclooctadienyl
(d) tertiary organo phosphorus
(e) tertiary organo arsenic

and mixtures of any of the above ligands.

3. A process according to claim 2 in which the rhodium source is selected from the group consisting of:

$HRh(CO)(PPh_3)_3$
$HRh(CO)(AsPh_3)_3$
$Rh(CO)_2(acac)$
$[Rh(PPh_3)_3]_2$
$HRh(Ph_2PCH_2CH_2PPh_2)_2$ and
$HRh(CO)[P(OPh)_3]_3$

4. A process according to claim 1 in which the mixed ligands X'R'' have the formula $PR^7R^8R^9$ and $AsR^{10}R^{11}R^{12}$ in which $R^7$ to $R^{12}$ may be alkyl, cycloalkyl, aryl, aralkyl, alkoxy, cycloalkoxy, aryloxy and aralkyloxy groups and may be the same or different.

5. A process according to claim 4 in which $R^7$ to $R^{12}$ are aryl and/or aryloxy groups.

6. A process according to claim 5 in which the mixed ligands are $AsPH_3$ and $PPh_3$.

7. A process according to claim 6 in which the $AsPH_3/PPh_3$ mol ratio is from 1:1 to 3:1.

8. A process according to claim 1 which comprises carrying out the reaction using a rhodium catalyst containing a tertiary organo phosphorus and tertiary organo arsenic represented by the general formula,

$$X'R''_3$$

wherein X' represents phosphorus or arsenic and R'' represents an organic group, and carbon monoxide in the presence of free mixed ligands comprising a tertiary organo phosphorus and tertiary organo arsenic represented by the general formula

$$X'R''_3$$

wherein X' and R'' have the same meaning as above, in excess of the coordination quantity for rhodium.

9. A process according to claim 8 wherein there is used as catalyst under hydroformylation conditions a rhodium compound containing both a tertiary organo phosphorus ligand and a tertiary organo arsenic ligand and having the general formula

$$HRh(CO)(ligand)_3$$

in the presence of excess of mixed ligands of a tertiary organo phosphorus compound and a tertiary organo arsenic compound.

10. A process according to claim 9 wherein there is used as catalyst a rhodium complex having the general formula

$$HRh(CO)PR^1R^2R^3)_n(AsR^4R^5R^6)_{3-n}$$

in which $R^1$, $R^2$, $R^3$, $R^4$, $R^5$ and $R^6$ are selected from the group consisting of alkyl, cycloalkyl, aryl and aralkyl and may be the same or different and n is 1 or 2, in the presence of excess mixed ligands of P and As having substituents selected from said group which may be the same as or different from those contained in the rhodium complex.

11. A process according to any one of the preceding claims in which the hydroformylation conditions comprise a temperature in the range of ambient to about 150°C, a total pressure in the range atmospheric to about 100 atmospheres and a $H_2/CO$ mol ratio of 10/1 to 1/10.

12. A process according to any one of the preceding claims in which a solvent is present.

13. A process according to claim 12 in which the solvent is selected from the group consisting of saturated hydrocarbons, aromatic hydrocarbons, oxygen-containing compounds and hydroformylation products and by-products.

**Patentansprüche**

1. Verfahren zur Herstellung eines Aldehyds durch Hydroformylierung eines Olefins, das nicht Octen-1 ist, mit Wasserstoff und Kohlenmonoxid in Gegenwart eines Rhodium enthaltenden Hydroformylierungskatalysatorsystems unter Bildung eines Aldehyds, der ein Kohlenstoffatom mehr als das Olefin enthält, dadurch gekennzeichnet, daß das Katalysatorsystem eine Rhodiumquelle und freie gemischte Liganden im Überschuß über die Menge, die zur Koordinierung des Rhodiumatoms erforderlich ist, umfaßt, wobei diese Liganden einen tertiären Organophosphor- und einen tertiären Organoarsenliganden der Formel $X'R''_3$, worin X' Phosphor oder Arsen und jedes R'' unabhängig eine organische Gruppe darstellen, umfassen und die gemischten tertiären Organophosphor- und tertiären Organoarsenliganden in einem Molverhältnis von 20:1 bis 1:20 vorliegen.

2. Verfahren gemaß Anspruch 1, worin die Rhodiumquelle ausgewählt ist aus der Gruppe bestehend aus Rhodiummetall, einer anorganischen Rhodiumverbindung, einem Rhodiumsalz einer organischen Säure und einem Rhodiumkomplex, der einen der folgenden Liganden enthält:

(a) CO
(b) Acetylacetonat
(c) Cyclooctadienyl
(d) tertiäres Organophosphor
(e) tertiäres Organoarsen

und Gemische aus irgendwelchen der vorstehenden Liganden.

3. Verfahren gemäß Anspruch 2, worin die Rhodiumquelle ausgewählt ist aus der Gruppe bestehend aus:

$HRh(CO)(PPh_3)_3$
$HRh(CO)(AsPh_3)_3$
$Rh(CO)_2(acac)$
$[Rh(PPh_3)_3]_2$
$HRh(Ph_2PCH_2CH_2PPh_2)_2$ und
$HRh(CO)[P(OPh)_3]_3$

4. Verfahren gemäß Anspruch 1, worin die gemischten Liganden X'R'' die Formeln $PR^7R^8R^9$ und $AsR^{10}R^{11}R^{12}$ aufweisen, worin $R^7$ bis $R^{12}$ Alkyl-, Cycloalkyl-, Aryl-, Aralkyl-, Alkoxy-, Cycloalkoxy-, Aryloxy- und Aralkyloxygruppen sein können und gleich oder verscheiden sein können.

5. Verfahren gemäß Anspruch 4, worin $R^7$ bis $R^{12}$ Aryl- und/oder Aryloxygruppen sind.

6. Verfahren gemäß Anspruch 5, worin die gemischten Liganden $AsPh_3$ und $PPh_3$ sind.

7. Verfahren gemäß Anspruch 6, worin das Molverhältnis $AsPh_3/PPh_3$ 1:1 bis 3:1 beträgt.

8. Verfahren gemäß Anspruch 1, welches darin besteht, daß man die Reaktion unter Verwendung eines Rhodiumkatalysators, der einem tertiären Organophosphor- und tertiären Organoarsenliganden der allgemeinen Formel $X'R''_3$, worin X' Phosphor oder Arsen bedeutet und $R^4$ eine organische Gruppe darstellt, und Kohlenmonoxid enthält, in Gegenwart von freien gemischten Liganden, die einen tertiären Organophosphor- und tertiaren Organoarsenliganden der allgemeinen Formel $X'R''_3$, worin X' und R'' die gleiche bedeutung wie vorstehend aufweisen, umfassen, im Überschuß über die Koordinationsmenge für Rhodium durchführt.

9. Verfahren gemäß Anspruch 8, worin als Katalysator unter Hydroformylierungsbedingungen eine Rhodiumverbindung, die sowohl einen tertiären Organophosphorliganden als auch einen tertiären Organoarsenliganden enthält und die allgemeine Formel $HRh(CO)(ligand)_3$ aufweist, in Gegenwart von überschlüssigen gemischten Liganden aus einer tertiären Organophosphorverbindung und einer tertiären Organoarsenverbindung verwendet wird.

10. Verfahren gemäß Anspruch 9, worin als Katalysator ein Rhodiumkomplex mit der allgemeinen Formel

$$HRh(CO)(PR^1R^2R^3)_n(AsR^4R^5R^6)_{3-n}$$

worin $R^1$, $R^2$, $R^3$, $R^4$, $R^5$ und $R^6$ ausgewählt sind aus der Gruppe bestehend aus Alkyl, Cycloalkyl, Aryl und Aralkyl und gleich oder verschieden sein können und n 1 oder 2 bedeutet, in Gegenwart von überschüssigen gemischten Liganden von P und As, die Substituenten ausgewählt aud dieser Gruppe die gleich oder verschieden von den in Rhodiumkomplex enthaltenen sein können, aufweisen, verwendet wird.

11. Verfahren gemäß einem der vorstehenden Ansprüche, worin die Hydroformylierungsbedingungen eine Temperatur im Bereich von Umgebungstemperatur bis etwa 150°C, einen Gesamtdruck im Bereich von Normaldruck bis etwa 100 at und eine Molverhältnis $H_2/CO$ von 10:1 bis 1:10 umfassen.

12. Verfahren gemäß einem der vorstehenden Ansprüche, worin ein Lösungsmittel vorhanden ist.

13. Verfahren gemäß Anspruch 12, worin das Lösungsmittel ausgewählt ist aus der Gruppe bestehend aus gesättigten Kohlenwasserstoffen, aromatische Kohlenwasserstoffen, sauerstoff enthaltenden Verbindungen und Hydroformylierungsprodukten und Nebenprodukten.

**Revendications**

1. Procédé de production d'un aldéhyde par hydroformylation d'une oléfine autre que l'octène-1 avec de l'hydrogène et de l'oxyde de carbone en présence d'un catalyseur d'hydroformylation contenant du rhodium pour former un aldéhyde ayant un atome de carbone de plus que l'oléfine, caractérisé en ce que le catalyseur comprend une source de rhodium et des coordinats mixtes libres en excès par rapport à la quantité nécessaire pour la coordination à l'atome de rhodium, lesdits coordinats comprenant un coordinat organophosphoré tertiaire et un coordinat organo-arsénié tertiaire de formule $X'R''_3$ dans laquelle X' est le phosphore ou l'arsenic et chaque R'' représente, indépendamment, un groupe organique, les coordinats organophosphoré et organo-arsénié tertiaires mixtes étant dans un rapport molaire de 20:1 à 1:20.

2. Procédé suivant la revendication 1, dans lequel la source de rhodium est choisie dans le groupe comprenant le rhodium métallique, un composé inorganique de rhodium, un sel de rhodium d'un acide organique et un complexe de rhodium contenant l'un quelconque des coordinats suivants:

(a) CO
(b) acétylacétonate
(c) cyclo-octadiényle
(d) coordinat organophosphoré tertiaire
(e) coordinat organo-arsénié tertiaire

et des mélanges en nombre quelconque des coordinats ci-dessus.

3. Procédé suivant la revendication 2, dans lequel la source de rhodium est choisie dans le groupe comprenant:

$HRh(CO)(PPh_3)_3$
$HRh(CO)(AsPh_3)_3$
$Rh(CO)_2(acac)$
$[Rh(PPh_3)_3]_2$
$HRh(Ph_2PCH_2CH_2PPh_2)_2$ et
$HRh(CO)[P(OPh)_3]_3$

4. Procédé suivant la revendication 1, dans lequel les coordinats mixtes $X'R''_3$ répondent à la formule $PR^7R^8R^9$ et à la formule $AsR^{10}R^{11}R^{12}$ dans lesquelles $R^7$ à $R^{12}$ peuvent êtres des groupes alkyle, cycloalkyle,

aryle, aralkyle, alkoxy, cycloalkoxy, aryloxy et aralkoxy et peuvent être égaux ou différents.

5. Procédé suivant la revendication 4, dans lequel $R^7$ à $R^{12}$ sont des groupes aryle et/ou aryloxy.

6. Procédé suivant la revendication 5, dans lequel les coordinats mixtes sont $AsPh_3$ et $PPh_3$.

7. Procédé suivant la revendication 6, dans lequel le rapport molaire $AsPh_3/PPh_3$ va de 1:1 à 3:1.

8. Procédé suivant la revendication 1, qui consiste à conduire la réaction en utilisant un catalyseur au rhodium contenant un coordinat organophosphoré tertiaire et un coordinat organo-arsénié tertiaire représentés par la formule générale:

$$X'R''_3$$

dans laquelle X' représente le phosphore ou l'arsenic et R'' représente un groupe organique, et l'oxyde de carbone en présence de coordinats mixtes libres comprenant un coordinat organophosphoré tertiaire et un coordinat organo-arsenié tertiaire représentés par la formule générale:

$$X'R''_3$$

dans laquelle X' et R'' ont les définitions données ci-dessus, en excès par rapport à la quantité de coordination vis-à-vis du rhodium.

9. Procédé suivant la revendication 8, dans lequel on utilise comme catalyseur dans les conditions d'hydroformylation un composé de rhodium contenant à la fois un coordinat organophosphoré tertiaire et un coordinat organo-arsénié tertiaire et répondant à la formule générale:

$$HRh(CO)(coordinat)_3$$

en présence d'un excès de coordinats mixtes d'un composé organophosphoré tertiaire et d'un composé organo-arsénié tertiaire.

10. Procédé suivant la revendication 9, dans lequel on utilise comme catalyseur un complexe de rhodium répondant à la formule générale:

$$HRh(Co)(PR^1R^2R^3)_n(AsR^4R^5R^6)_{3-n}$$

dans laquelle $R^1$, $R^2$, $R^3$, $R^4$, $R^5$ et $R^6$ sont choisis entre des groupes alkyle, cycloalkyle, aryle et aralkyle et peuvent être égaux ou différents et *n* est égal à 1 ou 2, en présence de coordinats mixtes de P et As en excès ayant des substituants choisis dans ledit groupé qui peuvent être égaux à, ou différents de, ceux qui sont contenus dans le complexe de rhodium.

11. Procédé suivant l'une quelconque des revendications précédentes, dans lequel les conditions d'hydroformylation impliquent un température comprise dans l'intervalle allant de la température ambiante à environ 150°C, une pression totale comprise dans l'intervalle allant de la pression atmosphérique à environ 100 atmosphères et un rapport molaire $H_2/CO$ de 10/1 à 1/10.

12. Procédé suivant l'une quelconque des revendications précédentes, dans lequel un solvent est présent.

13. Procédé suivant la revendication 12, dans lequel le solvant est choisi dans le groupe comprenant des hydrocarbures saturés, des hydrocarbures aromatiques, des composés contenant de l'oxygène et des produits et sous-produits d'hydroformylation.